# EUROPEAN PATENT APPLICATION

(11) **EP 2 329 823 A1**
(43) Date of publication of application: **08.06.2011**
(21) Application number: 09811518.1
(22) Date of filing: 02.09.2009
(51) Int. Cl.: A61K 31/519, A61K 9/16, A61K 9/30, A61K 9/32, A61K 9/36, A61K 47/12, A61K 47/32, A61K 47/38, A61P 13/08, A61P 15/00, A61P 15/08, A61P 15/18, A61P 17/00, A61P 35/00, A61P 43/00

(54) **METHOD FOR IMPROVING ABSORBABILITY OF PREPARATION, AND PREPARATION HAVING IMPROVED ABSORBABILITY**

(30) Priority: 03.09.2008 JP 2008226502
(71) Applicant: Takeda Pharmaceutical Company Limited, Osaka-shi, Osaka 541-0045 (JP)
(72) Inventor: KAMIKAWA, Kazuhisa, Osaka-shi Osaka 532-8686 (JP); OKABE, Takayuki, Osaka-shi Osaka 532-8686 (JP); NAKAMURA, Miho, Osaka-shi Osaka 532-8686 (JP)
(74) Representative: König, Gregor Sebastian
(86) International application number: PCT/JP2009/065352
(87) International publication number: WO 2010/026993

(57) **Abstract**

The present invention provides a method for improving absorbability of the active ingredient of an enteric polymer coated preparation, which includes using an organic acid.

## Description

### Technical Field

The present invention relates to a method for improving absorbability of a preparation, and a preparation with improved absorbability.

### (Background of the Invention)

Many preparations containing an active ingredient and alkali, which are coated with an enteric film, are known (patent documents 1 to 17).

In addition, as a compound showing a gonadotropin releasing hormone antagonizing activity, a compound chemical-structurally characterized in that the para-position of a phenyl group at the 6-position of the thieno[2,3-d]pyrimidine skeleton is substituted by 3-C₁₋₄ alkoxyureido is known (patent document 18).

### [Document List]

### [patent documents]

patent document 1: US Patent No. 4539198
patent document 2: US Patent No. 5711967
patent document 3: WO98/27967
patent document 4: WO2001/058424
patent document 5: US-A-2005/025824
patent document 6: US-A-2004/028737
patent document 7: WO2004/108067
patent document 8: WO2004/096208
patent document 9: WO2005/041934
patent document 10: US-A-2005/118256
patent document 11: WO2005/055955
patent document 12: WO2005/072709
patent document 13: WO2005/077420
patent document 14: WO2005/099666
patent document 15: WO2005/105036
patent document 16: WO2005/105045
patent document 17: WO2006/014973
patent document 18: WO2004/067535

### [SUMMARY OF THE INVENTION]

### Problems to be Solved by the Invention

It is a problem of the present invention to improve, in an enteric polymer-coated preparation comprising, as an active ingredient, a compound chemical-structurally characterized in that the para-position of a phenyl group at the 6-position of the thieno[2,3-d]pyrimidine skeleton is substituted by 3-C₁₋₄ alkoxyureido, oral absorbability of the active ingredient and increase stability thereof.

### Means of Solving the Problems

The present inventors have conducted intensive studies in an attempt to solve the aforementioned problem and found that the oral absorbability of the active ingredient can be improved by adding an organic acid, which enhances the solubility of the active ingredient, and further that the stability of the active ingredient can be increased by separating the organic acid and the active ingredient in the preparation (forming an intermediate layer between organic acid layer and active ingredient layer), which resulted in the completion of the present invention.
Accordingly, the present invention relates to
[1] a preparation with improved absorbability of an active ingredient, comprising, as the active ingredient, a compound represented by the formula (I):

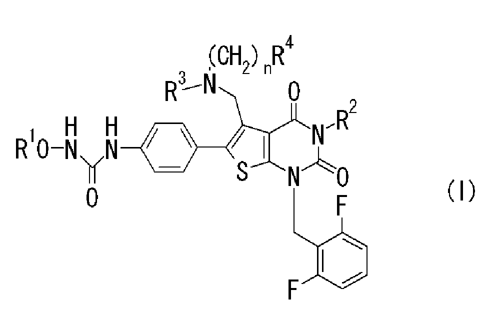

wherein
R¹ is C₁₋₄ alkyl;
R² is
(1) C₁₋₆ alkyl optionally having substituent(s) selected from the group consisting of (1') a hydroxy group, (2') C₁₋₄ alkoxy, (3') C₁₋₄ alkoxy-carbonyl, (4') di-C₁₋₄ alkyl-carbamoyl, (5') a 5- to 7-membered nitrogen-containing heterocyclic group, (6') C₁₋₄ alkyl-carbonyl and (7') halogen,
(2) C₃₋₈ cycloalkyl optionally having (1') a hydroxy group or (2') mono-C₁₋₄ alkyl-carbonylamino,
(3) a 5- to 7-membered nitrogen-containing heterocyclic group optionally having substituent(s) selected from the group consisting of (1') halogen, (2') a hydroxy group, (3') C₁₋₄ alkyl and (4') C₁₋₄ alkoxy,
(4) phenyl optionally having substituent(s) selected from the group consisting of (1') halogen, (2') C₁₋₄ alkoxy-C₁₋₄ alkyl, (3') mono-C₁₋₄ alkyl-carbamoyl-C₁₋₄ alkyl, (4') C₁₋₄ alkoxy and (5') mono-C₁₋₄ alkylcarbamoyl-C₁₋₄ alkoxy or
(5) C₁₋₄ alkoxy;
R³ is C₁₋₄ alkyl;
R⁴ is
(1) a hydrogen atom,
(2) C₁₋₄ alkoxy,
(3) C₆₋₁₀ aryl,
(4) N-C₁₋₄ alkyl-N-C₁₋₄ alkylsulfonylamino,
(5) a hydroxy group or
(6) a 5- to 7-membered nitrogen-containing heterocyclic group optionally having substituent(s) selected from the group consisting of (1') oxo, (2') C₁₋₄ alkyl, (3') hydroxy-C₁₋₄ alkyl, (4') C₁₋₄ alkoxy-carbonyl, (5') mono-C₁₋₄ alkyl-carbamoyl and (6') C₁₋₄ alkylsulfonyl;
n is an integer of 1 to 4;
provided that when R² is phenyl optionally having substituent(s), then R⁴ is a 5- to 7-membered nitrogen-containing heterocyclic group optionally having substituent(s) selected from the group consisting of (1) oxo, (2) hydroxy-C₁₋₄ alkyl, (3) C₁₋₄ alkoxy-carbonyl, (4) mono-C₁₋₄ alkyl-carbamoyl and (5) C₁₋₄ alkylsulfonyl; or a salt thereof, and an organic acid,
[2] the preparation of the above-mentioned [1], wherein the organic acid is one or more kinds selected from the group consisting of fumaric acid, citric acid, adipic acid, ascorbic acid, benzoic acid, oleic acid, succinic acid, acetic acid, tartaric acid, sorbic acid, lactic acid, maleic acid, malonic acid and malic acid,
[3] the preparation of the above-mentioned [1], wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea,
[4] the preparation of the above-mentioned [1], wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea or a salt thereof,
[5] the preparation of the above-mentioned [1], wherein a core comprising the active ingredient and the organic acid is coated with an enteric polymer,
[6] the preparation of the above-mentioned [1], wherein a core comprising the active ingredient is coated with a first layer comprising the organic acid, and further with a second layer comprising an enteric polymer,
[7] the preparation of the above-mentioned [1], wherein the amount of the organic acid is 0.1 - 70% (W/W), and the amount of the active ingredient is 0.01 - 90% (W/W), each relative to the weight of the preparation,
[8] a method for improving absorbability of a compound represented by the formula (I):

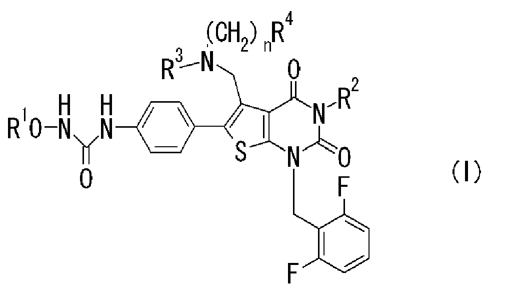

wherein
R¹ is C₁₋₄ alkyl;
R² is
(1) C₁₋₆ alkyl optionally having substituent(s) selected from the group consisting of (1') a hydroxy group, (2') C₁₋₄ alkoxy, (3') C₁₋₄ alkoxy-carbonyl, (4') di-C₁₋₄ alkyl-carbamoyl, (5') a 5- to 7-membered nitrogen-containing heterocyclic group, (6') C₁₋₄ alkyl-carbonyl and (7') halogen,
(2) C₃₋₈ cycloalkyl optionally having (1') a hydroxy group or (2') mono-C₁₋₄ alkyl-carbonylamino,
(3) a 5- to 7-membered nitrogen-containing heterocyclic group optionally having substituent(s) selected from the group consisting of (1') halogen, (2') a hydroxy group, (3') C₁₋₄ alkyl and (4') C₁₋₄ alkoxy,
(4) phenyl optionally having substituent(s) selected from the group consisting of (1') halogen, (2') C₁₋₄ alkoxy-C₁₋₄ alkyl, (3') mono-C₁₋₄ alkyl-carbamoyl-C₁₋₄ alkyl, (4') C₁₋₄ alkoxy and (5') mono-C₁₋₄ alkylcarbamoyl-C₁₋₄ alkoxy or
(5) C₁₋₄ alkoxy;
R³ is C₁₋₄ alkyl;
R⁴ is
(1) a hydrogen atom,
(2) C₁₋₄ alkoxy,
(3) C₆₋₁₀ aryl,
(4) N-C₁₋₄ alkyl-N-C₁₋₄ alkylsulfonylamino,
(5) a hydroxy group or
(6) a 5- to 7-membered nitrogen-containing heterocyclic group optionally having substituent(s) selected from the group consisting of (1') oxo, (2') C₁₋₄ alkyl, (3') hydroxy-C₁₋₄ alkyl, (4') C₁₋₄ alkoxy-carbonyl, (5') mono-C₁₋₄ alkyl-carbamoyl and (6') C₁₋₄ alkylsulfonyl;
n is an integer of 1 to 4;
provided that when R² is phenyl optionally having substituent(s), then R⁴ is a 5- to 7-membered nitrogen-containing heterocyclic group optionally having substituent(s) selected from the group consisting of (1) oxo, (2) hydroxy-C₁₋₄ alkyl, (3) C₁₋₄ alkoxy-carbonyl, (4) mono-C₁₋₄ alkyl-carbamoyl and (5) C₁₋₄ alkylsulfonyl; or a salt thereof, which is an active ingredient of an enteric polymer-coated preparation, which method comprises using an organic acid,
[9] the method of the above-mentioned [8], wherein the organic acid is one or more kinds selected from the group consisting of fumaric acid, citric acid, adipic acid, ascorbic acid, benzoic acid, oleic acid, succinic acid, acetic acid, tartaric acid, sorbic acid, lactic acid, maleic acid, malonic acid and malic acid,
[10] the method of the above-mentioned [8], wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea,
[11] the method of the above-mentioned [8], wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea, or a salt thereof,
[12] the method of the above-mentioned [8], wherein the enteric polymer-coated preparation is a preparation comprising a core comprising the active ingredient and the organic acid, which is coated with the enteric polymer,
[13] the method of the above-mentioned [8], wherein the enteric polymer-coated preparation is a preparation comprising a core comprising the active ingredient, which is coated with a first layer comprising the organic acid, and further with a second layer comprising the enteric polymer, and the like.

### Effect of the Invention

The method of the present invention can improve absorbability of the active ingredient in an enteric polymer-coated preparation. As a result, the amount of the active ingredient absorbed by the body can be increased as compared to conventional preparations. The increase in the absorbed amount is 1.01- to 100-fold, preferably 1.05- to 30-fold, more preferably 1.1- to 10-fold, based on the absorption amount of the preparation before improvement as 1.

### (Detailed Description of the Invention)

The present invention is explained in detail in the following.
In the present specification, examples of the "organic acid" include edible organic acids such as adipic acid, ascorbic acid, benzoic acid, oleic acid, succinic acid, acetic acid, tartaric acid, sorbic acid, fumaric acid, lactic acid, maleic acid, malonic acid, citric acid, malic acid and the like.
In addition, organic acids such as formic acid, trifluoroacetic acid, phthalic acid, oxalic acid, methanesulfonic acid, benzenesulfonic acid, p-toluenesulfonic acid and the like can also be used. Of these, fumaric acid and citric acid are particularly preferable.

The amount of the organic acid is desirably 0.1 - 70%(W/W), preferably 0.5 - 60%(W/W), more preferably 1 - 50%(W/W), relative to the weight of the preparation.

In the present specification, the "enteric polymer-coated preparation" means a preparation wherein a core containing an active ingredient is coated with an enteric polymer (powder, tablet, granule, pill etc.).
The "enteric polymer-coated preparation" means
(a) a preparation obtained by coating a core containing an active ingredient and an organic acid with an enteric polymer, or
(b) a preparation obtained by coating a core containing an active ingredient with a first layer containing an organic acid, and further with a second layer containing an enteric polymer.

In the present specification, examples of the "enteric polymer" include one or more compounds selected from carboxymethylethylcellulose, dried methacrylic acid copolymer LD, cellulose acetate phthalate, shellac, cellulose acetate phthalate, hydroxypropylmethylcellulose acetate succinate, hydroxypropylmethylcellulose phthalate, polyvinyl acetate phthalate, methacrylic acid copolymer L, methacrylic acid copolymer LD, methacrylic acid copolymer S and the like. Of these, one or more compounds selected from methacrylic acid copolymer L (Eudragit (registered trade mark), L100), methacrylic acid copolymer S (Eudragit (registered trade mark), S100), methacrylic acid copolymer LD, dried methacrylic acid polymer LD and the like are preferable.
The amount of the enteric polymer to be coated is desirably 1 - 200%(W/W), preferably 5 - 100% (W/W), more preferably 10 - 60%(W/W), relative to the weight of the core granule.

The "active ingredient" in the present specification means a compound having a pharmacological action.
The "active ingredient" in the present specification is desirably a compound represented by the formula (I):

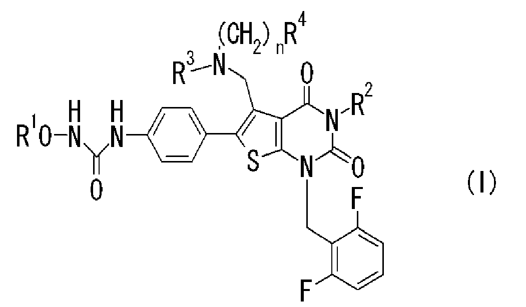

wherein
R¹ is C₁₋₄ alkyl;
R² is
(1) C₁₋₆ alkyl optionally having substituent(s) selected from the group consisting of (1') a hydroxy group, (2') C₁₋₄ alkoxy, (3') C₁₋₄ alkoxy-carbonyl, (4') di-C₁₋₄ alkyl-carbamoyl, (5') a 5- to 7-membered nitrogen-containing heterocyclic group, (6') C₁₋₄ alkyl-carbonyl and (7') halogen,
(2) C₃₋₈ cycloalkyl optionally having (1') a hydroxy group or (2') mono-C₁₋₄ alkyl-carbonylamino,
(3) a 5- to 7-membered nitrogen-containing heterocyclic group optionally having substituent(s) selected from the group consisting of (1') halogen, (2') a hydroxy group, (3') C₁₋₄ alkyl and (4') C₁₋₄ alkoxy,
(4) phenyl optionally having substituent(s) selected from the group consisting of (1') halogen, (2') C₁₋₄ alkoxy-C₁₋₄ alkyl, (3') mono-C₁₋₄ alkyl-carbamoyl-C₁₋₄ alkyl, (4') C₁₋₄ alkoxy and (5') mono-C₁₋₄ alkylcarbamoyl-C₁₋₄ alkoxy or
(5) C₁₋₄ alkoxy;
R³ is C₁₋₄ alkyl;
R⁴ is
(1) a hydrogen atom,
(2) C₁₋₄ alkoxy,
(3) C₆₋₁₀ aryl,
(4) N-C₁₋₄ alkyl-N-C₁₋₄ alkylsulfonylamino,
(5) a hydroxy group or
(6) a 5- to 7-membered nitrogen-containing heterocyclic group optionally having substituent(s) selected from the group consisting of (1') oxo, (2') C₁₋₄ alkyl, (3') hydroxy-C₁₋₄ alkyl, (4') C₁₋₄ alkoxy-carbonyl, (5') mono-C₁₋₄ alkyl-carbamoyl and (6') C₁₋₄ alkylsulfonyl;
n is an integer of 1 to 4;
provided that when R² is phenyl optionally having substituent(s), then R⁴ is a 5- to 7-membered nitrogen-containing heterocyclic group optionally having substituent(s) selected from the group consisting of (1) oxo, (2) hydroxy-C₁₋₄ alkyl, (3) C₁₋₄ alkoxy-carbonyl, (4) mono-C₁₋₄ alkyl-carbamoyl and (5) C₁₋₄ alkylsulfonyl; which is described in W02004/067535 and is chemical-structurally characterized in that the para-position of a phenyl group at the 6-position of the thieno[2,3-d]pyrimidine skeleton is substituted by 3-C₁₋₄ alkoxyureido (hereinafter to be sometimes abbreviated as compound (I)) or a salt thereof.

The definition of each term is described in the following paragraphs.
Examples of the "C₁₋₄ alkyl" include a linear C₁₋₄ alkyl (e.g. methyl, ethyl, propyl, butyl, and the like), a branched C₃₋₄ alkyl (e.g., isopropyl, isobutyl, sec-butyl, tert-butyl, and the like), and the like.
Examples of the "C₁₋₆ alkyl" include a linear C₁₋₆ alkyl (e.g. methyl, ethyl, propyl, butyl, pentyl, hexyl and the like), a branched C₃₋₆ alkyl (e.g., isopropyl, isobutyl, sec-butyl, tert-butyl, isopentyl and the like), and the like.
Examples of the "C₁₋₄ alkoxy" include a linear C₁₋₄ alkoxy (e.g. methoxy, ethoxy, propoxy, butoxy, and the like), a branched C₃₋₄ alkoxy (e.g., isopropoxy, isobutoxy, sec-butoxy, tert-butoxy, and the like), and the like.
Examples of the "C₁₋₄ alkoxy-carbonyl" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, and the like.
Examples of the "di-C₁₋₄ alkyl-carbamoyl" include dimethylcarbamoyl, diethylcarbamoyl, dipropylcarbamoyl, diisopropylcarbamoyl, N-ethyl-N-methylcarbamoyl, and the like.

Examples of the "5- to 7-membered nitrogen-containing heterocyclic group" include pyrrolidin-1-yl, pyrrolidin-2-yl, pyrrolidin-3-yl, oxazolidin-3-yl, thiazolidin-3-yl, isoxazolidin-2-yl, isothiazolidin-2-yl, imidazolidin-1-yl, imidazolidin-2-yl, imidazolidin-4-yl, pyrazolidin-1-yl, pyrazolidin-3-yl, pyrazolidin-4-yl, pyrrol-1-yl, pyrrol-2-yl, pyrrol-3-yl, imidazol-1-yl, imidazol-2-yl, imidazol-4-yl, pyrazol-1-yl, pyrazol-3-yl, pyrazol-4-yl, 1,2,3-triazol-1-yl, 1,2,5-triazol-1-yl, tetrazol-1-yl, tetrazol-2-yl, tetrazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, piperidin-1-yl, piperidin-2-yl, piperidin-3-yl, piperidin-4-yl, piperazin-1-yl, piperazin-2-yl, morpholin-2-yl, morpholin-3-yl, morpholin-4-yl, pyridin-2-yl, pyridin-3-yl, pyridin-4-yl, pyrazin-2-yl, pyrimidin-2-yl, pyrimidin-4-yl, pyrimidin-5-yl, pyridazin-3-yl, pyridadin-4-yl, and the like. Among them, preferable examples are pyrrolidin-1-yl, pyrrolidin-2-yl, imidazol-1-yl, imidazol-2-yl, 1,2,3-triazol-1-yl, 1,2,5-triazol-1-yl, tetrazol-1-yl, tetrazol-2-yl, pyridin-2-yl, pyridin-4-yl, and the like.

Examples of the "C₁₋₄ alkyl-carbonyl" include methyl-carbonyl, ethyl-carbonyl, propyl-carbonyl, isopropyl-carbonyl, butyl-carbonyl, isobutyl-carbonyl, sec-butyl-carbonyl, tert-butyl-carbonyl, and the like.
The "halogen" includes fluorine, chlorine, bromine and iodine.
Examples of the "mono-C₁₋₄ alkyl-carbonylamino" include methylcarbonylamino, ethylcarbonylamino, propylcarbonylamino, isopropylcarbonylamino, butylcarbonylamino, isobutylcarbonylamino, sec-butylcarbonylamino, tert-butylcarbonylamino, and the like.
Examples of the "C₃₋₈ cycloalkyl" include cyclopropyl, cyclobutyl, cyclopentyl, cyclohexyl, cycloheptyl, cyclooctyl, and the like.
The "C₁₋₄ alkoxy-C₁₋₄ alkyl" includes methoxymethyl, 1-methoxyethyl, 2-methoxyethyl, 1-methoxypropyl, 2-methoxypropyl, 3-methoxypropyl, 1-methoxybutyl, 2-methoxybutyl, 3-methoxybutyl, 4-methoxybutyl, 1-methoxy-1-methylethyl, 2-methoxy-1-methylethyl, 1-methoxy-1-methylpropyl, 2-methoxy-1-methylpropyl, 3-methoxy-1-methylpropyl, 1-(methoxymethyl)propyl, 1-methoxy-2-methylpropyl, 2-methoxy-2-methylpropyl, 3-methoxy-2-methylpropyl, 2-methoxy-1,1-dimethylethyl, ethoxymethyl, 2-ethoxyethyl, 3-ethoxypropyl, 4-ethoxybutyl, and the like.

Examples of the "mono-C₁₋₄ alkyl-carbamoyl-C₁₋₄ alkyl" include methylaminocarbonylmethyl, ethylaminocarbonylmethyl, 2-methylaminocarbonylethyl, 2-ethylaminocarbonylethyl, and the like.
Examples of the "mono-C₁₋₄ alkyl-carbamoyl-C₁₋₄ alkoxy" include methylaminocarbonylmethoxy, ethylaminocarbonylmethoxy, 2-methylaminocarbonylethoxy, 2-ethylaminocarbonylethoxy, and the like.
Examples of the "C₆₋₁₀ aryl" include phenyl, 1-naphthyl, 2-naphthyl, and the like.
Examples of the "N-C₁₋₄ alkyl-N-C₁₋₄ alkylsulfonylamino" include N-methyl-N-methylsulfonylamino, N-ethyl-N-methylsulfonylamino, N-ethylsulfonyl-N-methylamino, N-ethyl-N-ethylsulfonylamino, and the like.
Examples of the "hydroxy-C₁₋₄ alkyl" include hydroxymethyl, 1-hydroxyethyl, 2-hydroxyethyl, 1-hydroxypropyl, 2-hydroxypropyl, 3-hydroxypropyl, 1-hydroxybutyl, 2-hydroxybutyl, 3-hydroxybutyl, 4-hydroxybutyl, 1-hydroxy-1-methylethyl, 2-hydroxy-1-methylethyl, 1-hydroxy-1-methylpropyl, 2-hydroxy-1-methylpropyl, 3-hydroxy-1-methylpropyl, 1-(hydroxymethyl)propyl, 1-hydroxy-2-methylpropyl, 2-hydroxy-2-methylpropyl, 3-hydroxy-2-methylpropyl, 2-hydroxy-1,1-dimethylethyl, and the like.
Examples of the "mono-C₁₋₄ alkyl-carbamoyl" include methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, and the like.
Examples of the "C₁₋₄ alkylsulfonyl" include methylsulfonyl, ethylsulfonyl, propylsulfonyl, isopropylsulfonyl, butylsulfonyl, isobutylsulfonyl, sec-butylsulfonyl, tert-butylsulfonyl, and the like.

As R¹, methyl and ethyl are preferable, and especially methyl is preferable.
As R², a 5- to 7-membered nitrogen-containing heterocyclic group optionally having substituent(s) selected from the group consisting of (1) a halogen, (2) a hydroxy group, (3) C₁₋₄ alkyl and (4) C₁₋₄ alkoxy is preferable. Among them, pyridyl (pyridin-2-yl, pyridin-3-yl, pyridin-4-yl) optionally having substituent(s) selected from the group consisting of (1) a halogen, (2) a hydroxy group, (3) C₁₋₄ alkyl and (4) C₁₋₄ alkoxy is more preferable. Especially, unsubstituted pyridin-2-yl is preferable.
As R³, methyl and ethyl are preferable. Especially, methyl is preferable.
As R⁴, a C₁₋₄ alkoxy is preferable. Especially, methoxy and ethoxy are preferable.
As n, 1 or 2 is preferable. Especially, 2 is preferable.
Preferable examples of the combination of R³, R⁴ and n include one wherein R³ is methyl, R⁴ is a hydrogen atom and n is 1.

Preferable examples of compound (I) include N-(4-(1-(2,6-difluorobenzyl)-5-(((2-methoxyethyl)(methyl)amino)methyl)-2,4-dioxo-3-(2-pyridinyl)-1,2,3,4-tetrahydrothieno[2,3-d]pyrimidin-6-yl)phenyl)-N'-methoxyurea, N-(4-(1-(2,6-difluorobenzyl)-5-(((2-ethoxyethyl)(methyl)amino)methyl)-2,4-dioxo-3-(2-pyridinyl)-1,2,3,4-tetrahydrothieno[2,3-d]pyrimidin-6-yl)phenyl)-N'-methoxyurea, N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea and N-(4-(1-(2,6-difluorobenzyl)-5-((dimethylamino)methyl)-3-(6-methoxypyridin-3-yl)-2,4-dioxo-1,2,3,4-tetrahydrothieno[2,3-d]pyrimidin-6-yl)phenyl)-N'-methoxyurea.

Salts of compound (I) are preferably physiologically acceptable acid-added salts. Such salts include, for example, salts with inorganic acids (e.g., hydrochloric acid, hydrobromic acid, nitric acid, sulfuric acid, phosphoric acid, etc.), salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), and the like. When compound (I) has an acidic group, it may be formed into a physiologically acceptable salt with an inorganic base (e.g., alkali metals and alkaline earth metals such as sodium, potassium, calcium, magnesium, etc.; ammonia, and the like) or an organic base (e.g., trimethylamine, triethylamine, pyridine, picoline, ethanolamine, diethanolamine, triethanolamine, dicyclohexylamine, N,N'-dibenzylethylenediamine, and the like).

For example, compound (I) can be produced according to the following production methods. Compounds illustrated in the following reaction schemes include their salts. Examples of the salts include the same salts as the salts of compound (I), etc. Compounds (I)-(IV) illustrated in the following reaction schemes may be formed into the acceptable salts depending on the reaction conditions.

### (Production Method 1)

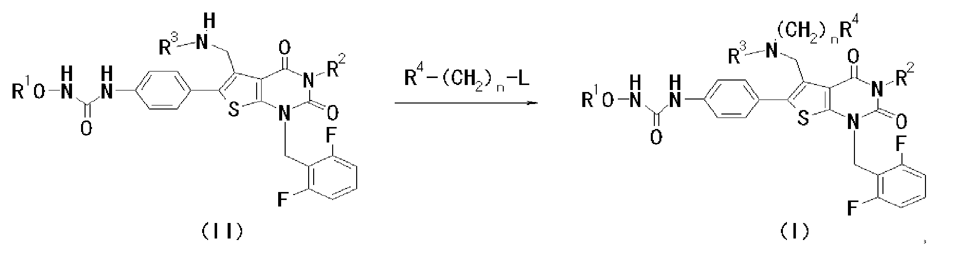

In the above formula, L is a leaving group, and other symbols are the same as defined above.

Examples of the "leaving group" represented by L include a halogen atom, C₁₋₄ alkylsulfonyloxy optionally having halogen atom(s), and the like. Examples of "C₁₋₄ alkylsulfonyloxy optionally having halogen atom(s)" include methanesulfonyloxy, ethanesulfonyloxy, trifluoromethanesulfonyloxy, and the like.

Compound (II) can be produced according to the methods disclosed in JP-A-2001-278884, WO 00/56739 or analogous methods thereto.
For example, compound (I) can be produced by reacting compound (II) and a compound represented by the formula: R⁴-(CH₂)ₙ-L. This reaction is preferably carried out in the presence of a base.
Examples of the "base" include inorganic bases such as sodium carbonate, sodium hydrogen carbonate, potassium carbonate, potassium hydrogen carbonate, sodium hydroxide, potassium hydroxide, thallium hydroxide, and the like; and organic bases such as triethylamine, diisopropylethylamine, pyridine, and the like.

The amount of the compound represented by the formula: R⁴-(CH₂)ₙ-L in the reaction of compound (II) with the compound represented by the formula: R⁴-(CH₂)ₙ-L is about 1 to about 3 moles per 1 mole of compound (II). The amount of a base is about 1 to about 3 moles per 1 mole of compound (II).

This reaction is usually carried out in a solvent inert to the reaction. Examples of "solvent" are an ether (e.g., diethyl ether, dioxane, dimethoxyethane, tetrahydrofuran, and the like), an aromatic hydrocarbon (e.g., benzene, toluene, and the like), an amide (e.g., dimethylformamide, dimethylacetamide, and the like), a halogenated hydrocarbon (e.g., chloroform, dichloromethane, and the like), and the like.
The reaction temperature is usually about 0 to about 150°C, preferably about 50 to about 80°C. The reaction time is usually about 1 to about 24 hours.

### (Production Method 2)

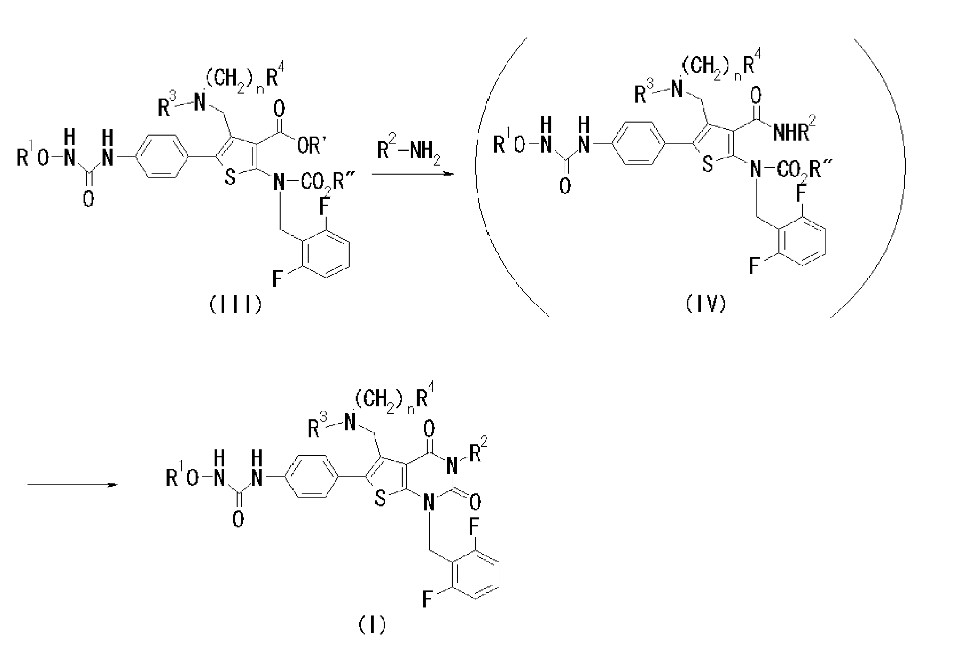

In the above formula, R' is a hydrogen atom or a C₁₋₄ alkyl; R" is a C₁₋₄ alkyl; and the other symbols are as defined above.

Examples of the C₁₋₄ alkyl represented by R' and R" are a linear C₁₋₄ alkyl (e.g., methyl, ethyl, propyl, butyl, and the like), a branched C₃₋₄ alkyl (e.g., isopropyl, isobutyl, sec-butyl, tert-butyl, and the like), and the like.

Compound (III) can be produced by any manner known *per se*, for example, by reacting p-nitrophenylacetone with a cyanoacetic ester compound and sulphur [e.g., Chem. Ber., 99, 94-100(1966)] followed by subjecting the obtained 2-amino-4-methyl-5-(4-nitrophenyl)thiophene to the methods disclosed in JP-A-9-169768, WO 96/24597 or analogous methods thereto.

1) When R' is hydrogen atom, compound (I) can be produced by reacting compound (III) with a compound represented by the formula: R²-NH₂ or a salt thereof in the presence of a condensing agent, to obtain compound (IV), following by subjecting to cyclization.

Examples of the "condensing agent" are WSC (1-ethyl-3-(3-dimethylaminopropyl)carbodiimide hydrochloride), DCC (dicyclohexylcarbodiimide), diethyl cyanophosphate, benzotriazol-1-yloxytripyrrolidinophosphonium hexafluorophosphate: PyBOP), and the like.
The amount of the "condensing agent" is about 1 to about 3 moles per 1 mole of compound (III).

This reaction is advantageously carried out in a solvent inert to the reaction.
Examples of the solvent are an alcohol (e.g., ethanol, methanol, and the like), an aromatic hydrocarbon (e.g., benzene, toluene, and the like), an amide (e.g., dimethylformamide, dimethylacetamide, and the like), a halogenated hydrocarbon (e.g., chloroform, dichloromethane, and the like), and so the like.
The reaction temperature is usually about 0 to about 150°C, preferably about 0 to about 25°C. The reaction time is usually about 1 to about 36 hours.
The product as produced in the manner mentioned above may be applied to the next reaction while it is still crude in the reaction mixture, or may be isolated from the reaction mixture in any ordinary manner.

Compound (IV) is subjected to cyclization in the presence of a base.
Examples of the "base" are inorganic bases such as sodium methoxide, sodium carbonate, sodium hydrogen carbonate, potassium carbonate, potassium hydrogen carbonate, sodium hydroxide, potassium hydroxide, thallium hydroxide; and organic bases such as triethylamine, pyridine, and the like.
The amount of the "base" is about 2 to about 20 moles, preferably about 5 to about 12 mole per 1 mole of compound (IV).

This reaction is usually carried out in a solvent inert to the reaction.
Examples of the solvent are an alcohol (e.g., ethanol, methanol, and the like), an aromatic hydrocarbon (e.g., benzene, toluene, and the like), an amide (e.g., dimethylformamide, dimethylacetamide, and the like), a halogenated hydrocarbon (e.g., chloroform, dichloromethane, and the like), and the like.
The reaction temperature is usually about 0 to about 150°C, preferably room temperature (about 15 to about 25°C). The reaction time is usually about 1 to about 36 hours.

2) When R' is an alkyl group, compound (I) can be produced by reacting compound (III) with an activated R²-NH₂.
   The activated R²-NH₂ can be produced in any *per se* known manner, for example, by reacting an organo-aluminum reagent with R²-NH₂ in a solvent inert to the reaction.
   Examples of the "organo-aluminum reagent" are trimethyl aluminum, dimethyl aluminum chloride, and the like; and a solution including them, and the like.
   The amount of the "organo-aluminum reagent" is about 1 to about 5 moles, preferably about 1 mole per 1 mole of R²-NH₂.
   Examples of the solvent are a halogenated hydrocarbon (e.g., chloroform, dichloromethane, and the like).
   The reaction temperature is usually about 0 to about 150°C, preferably about 0 to about 25°C. The reaction time is usually about 1 to about 6 hours.
   The cyclization can be carried out by reacting compound (III) with an activated R²-NH₂ to obtain compound (I).
   The amount of "compound (III)" is about 1/5 volume of a mixture of R²-NH₂ and the organo-aluminum reagent.
   This reaction is usually carried out in a solvent inert to the reaction.
   Such solvent is the same as those used in the reaction to obtain an activated R²-NH₂.
   The reaction temperature is usually about 0 to about 150°C, preferably about 0 to 25°C. The reaction time is usually about 1 to about 48 hours.
   Compound (I) can also be produced by a known hydrolysis reaction, deprotection reaction, acylation reaction, alkylation reaction, oxidation reaction, cyclization reaction, carbon chain expanding reaction, substituent exchanging reaction, or a combination thereof.

Compound (I) may be isolated and purified by *per se* known means of separation such as recrystallization, distillation and chromatography, and the like.
When compound (I) is obtained in free form, it can be converted to a salt by *per se* known methods or methods analogous thereto. When compound (I) is obtained in a salt form, it can be converted to the free form or another salt by *per se* known methods or methods analogous thereto.
Compound (I) may be a hydrate or a non-hydrate. The hydrate is exemplified by monohydrate, sesquihydrate and dihydrate.
When compound (I) is obtained as a mixture of optically active isomers, it can be resolved into the (R)- and (S)-forms by the conventional optical resolution techniques.
Compound (I) can be used as a prodrug. The prodrug of compound (I) or a salt thereof means a compound which is converted to compound (I) under physiological conditions or with a reaction due to an enzyme, an gastric acid, and the like in the living body, that is, a compound which is enzymatically converted to compound (I) with oxidation, reduction, hydrolysis, and the like; a compound which is converted to compound (I) with hydrolysis etc. by gastric acid, etc. The prodrug for compound (I) may, for example, be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy in compound (I) to an acetylation, palmitoylation, propanoylation, pivaloylation, succinylation, fumarylation, alanylation, dimethylaminomethylcarbonylation, etc.) and the like. Any of these compounds can be produced from compound (I) by a method known *per se*.
A prodrug of compound (I) may also be one which is converted into the compound of the present invention under a physiological condition, such as those described in "IYAKUHIN no KAIHATSU (Development of Pharmaceuticals)", Vol.7, Design of Molecules, p.163-198, Published by Hirokawa Shoten (1990).
Compound (I) may be labeled with an isotope (e.g., ³H, ¹⁴C, ³⁵S) and the like.

In the reaction described above, a starting compound having an amino group, a carboxy group or a hydroxy group as its substituent may be present as a compound in which a protective group employed ordinarily in a peptide chemistry has been introduced into such a substituent, and an object compound can be obtained by deprotection if necessary after the reaction.
A protective group for an amino group may, for example, be an optionally substituted C₁₋₆ alkyl-carbonyl (e.g., acetyl, propionyl, and the like), formyl, phenylcarbonyl, a C₁₋₆ alkyloxycarbonyl (e.g., methoxycarbonyl, ethoxycarbonyl, tert-butoxycarbonyl, and the like), phenyloxycarbonyl, a C₇₋₁₄ aralkyloxy-carbonyl (e.g., benzyloxycarbonyl, 9-fluorenylmethoxycarbonyl, and the like), trityl, phthaloyl and the like. Its substituent may, for example, be a halogen atom (e.g., fluorine, chlorine, bromine and iodine), a C₁₋₆ alkylcarbonyl (e.g., acetyl, propionyl, butyryl, and the like), nitro and the like, and the number of the substituents may be 1 to 3.
A protective group for a carboxy may, for example, be an optionally substituted C₁₋₆ alkyl (e.g., methyl, ethyl, propyl, isopropyl, butyl, t-butyl, and the like), phenyl, trityl, silyl and the like. Its substituent may, for example, be a halogen atom (e.g., fluorine, chlorine, bromine and iodine), a C₁₋₆ alkyl-carbonyl (e.g., acetyl, propionyl, butyryl, and the like), formyl, nitro, and the number of the substituents may be 1 to 3.
A protective group for a hydroxy group may, for example, be an optionally substituted C₁₋₆ alkyl (e.g., methyl, ethyl, propyl, isopropyl, butyl, tert-butyl, and the like), phenyl, a C₇₋₁₀ aralkyl (e.g., benzyl, and the like), a C₁₋₆ alkyl-carbonyl (e.g., acetyl, propionyl, and the like), formyl, phenyloxycarbonyl, a C₇₋₁₀ aralkyl-oxycarbonyl (e.g., benzyloxycarbonyl, and the like), tetrahydropyranyl, tetrahydrofuranyl, silyl and the like. Its substituent may, for example, be a halogen atom (e.g., fluorine, chlorine, bromine and iodine), a C₁₋₆ alkyl, phenyl, a C₇₋₁₁ aralkyl, nitro, and the like, and the number of the substituents may be 1 to 4.

The method for introducing and removing the protective group is demonstrated in accordance with a known method or analogous method thereof (e.g., the method described in Protective Groups in Organic Chemistry (J.F.W. McOmie et al, Plenum Press). A deprotection method may be a treatment with an acid, base, reduction, UV, hydrazine, phenylhydrazine, sodium N-methyldithiocarbamate, tetrabutylammonium fluoride, palladium acetate and the like.

Compound (I) or a salt thereof possesses excellent GnRH-antagonizing activity and is low in toxicity (e.g., acute toxicity, chronic toxicity, genetic toxicity, reproduction toxicity, cardiotoxicity, drug interaction, carcinogenicity). In addition, it is excellent in oral absorbability, action sustainability, stability and pharmacokinetics. Also, it is scarcely influenced by plasma ingredients. The compound of the present invention can therefore be safely used in a mammal (e.g., human, monkey, bovine, horse, dog, cat, rabbit, rat, mouse, etc.) for the preventing and/or treating diseases depending on male or female hormones, diseases due to excess of these hormones, etc., by suppressing gonadotropin secretion with its GnRH receptor-antagonizing action to control plasma sex hormone concentrations.

For example, compound (I) or a salt thereof is useful for preventing and/or treating sex hormone-dependent cancers (e.g., prostatic cancer, uterine cancer, breast cancer, pituitary tumor, etc.), bone metastasis of sex hormone-dependent cancer, prostatic hypertrophy, uterine fibroid, endometriosis, metrofibroma, precocious puberty, amenorrhea, premenstrual syndrome, dysmenorrhea, multilocular ovary syndrome, polycystic ovary syndrome, acne, alopecia, Alzheimer's disease (Alzheimer's disease, senile dementia of Alzheimer type and a mixed type thereof), and the like. Compound (I) or a salt thereof is also useful for the regulation of reproduction in males and females (e.g., pregnancy regulators, menstruation cycle regulators, etc.). Compound (I) or a salt thereof can also be used as a male or female contraceptive, or as a female ovulation inducer. Based on its rebound effect after withdrawal, compound (I) or a salt thereof can be used to treat infertility. Compound (I) or a salt thereof can be used as an agent for preventing and/or treating benign or malignant tumor which is hormone independent and LH-RH sensitive or hot flash. Compound (I) or a salt thereof can be used as an agent for preventing and/or treating irritable bowel syndrome and for preventing postoperative recurrence of sex hormone-dependent cancer (an agent for preventing postoperative recurrence of prostatic cancer; an agent for preventing postoperative recurrence of breast cancer or ovarian cancer in the condition before or after menopause; especially, an agent for preventing postoperative recurrence of breast cancer or ovarian cancer in the condition before menopause).
In addition, compound (I) or a salt thereof is useful for regulation of animal estrus, improvement of meat quality and promotion of animal growth in the field of animal husbandry. Compound (I) or a salt thereof is also useful as a fish spawning promoter.

Compound (I) or a salt thereof can also be used to suppress the transient rise in plasma testosterone concentration (flare phenomenon) observed in administration of a GnRH super-agonist such as leuprorelin acetate. Compound (I) or a salt thereof can be used in combination with a GnRH super-agonist such as leuprorelin acetate, gonadorelin, buserelin, triptorelin, goserelin, nafarelin, histrelin, deslorelin, meterelin, lecirelin, and the like. Among others, preferred is leuprorelin acetate.

It is also beneficial to use compound (I) or a salt thereof in conjunction with at least one member selected from the steroidal or nonsteroidal antiandrogen or antiestrogen, chemotherapeutic agent, GnRH antagonistic peptide, α-reductase inhibitor, α-receptor inhibitor, aromatase inhibitor, 17β-hydroxysteroid dehydrogenase inhibitor, adrenal androgen production inhibitor, kinase inhibitor, drug for hormone therapy, and drug inhibiting the action of a cell growth factor or its receptor, among others.

The "chemotherapeutic agent" mentioned above includes ifosfamide, adriamycin, peplomycin, cisplatin, cyclophosphamide, 5-FU, UFT, methotrexate, mitomycin C, mitoxantrone, etc.

The "GnRH antagonistic peptide" mentioned above includes non-oral GnRH antagonistic peptides such as cetrorelix, ganirelix, abarelix, etc.

The "adrenal androgen production inhibitor" mentioned above includes lyase (C₁₇, ₂₀-lyase) inhibitors, etc.

The "kinase inhibitor" mentioned above includes tyrosine kinase inhibitor, etc.

The "drugs for hormone therapy" includes antiestrogens, progesterons (e.g., MPA, etc.), androgens, estrogens and androgen antagonists, among others.

The "cell growth factor" may be any substance that promotes proliferation of cells and generally includes peptides with molecular weights not over 20,000 which express the action at low concentrations through binding to receptors. Specifically, there can be mentioned (1) EGF (epidermal growth factor) or substances having the substantially the same activity (e.g., EGF, heregulin (HER2 ligand), etc.), (2) insulin or substances having substantially the same activity (e.g., insulin, IGF (insulin-like growth factor)-1, IGF-2, etc.), (3) FGF (fibroblast growth factor) or substances having substantially the same activity (aFGF, bFGF, KGF (keratinocyte growth factor), HGF (hepatocyte growth factor), FGF-10, etc.), and (4) other cell growth factors (e.g., CSF (colony stimulating factor), EPO (erythropoietin), IL-2 (interleukin-2), NGF (nerve growth factor), PDGF (platelet-derived growth factor) and TGF β (transforming growth factor β), etc.), among others.

The "cell growth factor receptor" may be any receptor capable of binding said cell growth factor, including EGF receptor, heregulin receptor (HER2), insulin receptor-1, insulin receptor-2, IGF receptor, FGF receptor-1, FGF receptor-2, etc.

The drug inhibiting the action of a cell growth factor mentioned above includes herceptin (anti-HER2 receptor antibody), among others.

The above drug inhibiting the action of a cell growth factor or its receptor includes herbimycin, PD153035 [e.g., Science, 265 (5175) p. 1093, (1994)], etc.

As a further class of drugs inhibiting the action of a cell growth factor or its receptor includes HER2 inhibitors. The HER2 inhibitor may be any substance that inhibits the activity of HER2 (e.g., phosphorylating activity), thus including an antibody, a low-molecular weight compound (synthetic or natural product), an antisense, an HER2 ligand, heregulin, and any of them as partially modified or mutated in structure. Moreover, it may be a substance which inhibits HER2 activity by inhibiting HER2 receptor (e.g. HER2 receptor antibody). The low-molecular weight compound having HER2 inhibiting activity includes, for example, the compound described in WO 98/03505, namely, 1-[3-[4-[2-((E)-2-phenylethenyl)-4-oxazolylmethoxy]phenyl]propyl]-1,2,4-triazole and the like.

For prostatic hypertrophy, examples of such combination includes compound (I) or a salt thereof in combination with the GnRH super-agonist, antiandrogen, antiestrogen, GnRH antagonistic peptide, α-reductase inhibitor, α-receptor inhibitor, aromatase inhibitor, 17β-hydroxysteroid dehydrogenase inhibitor, adrenal androgen production inhibitor, kinase inhibitor, or the like.

For prostatic cancer, examples of such combination includes compound (I) or a salt thereof in combination with the GnRH super-agonist, antiandrogen, antiestrogen, chemotherapeutic agent (e.g., ifosfamide, UFT, adriamycin, peplomycin, cisplatin, etc.), GnRH antagonistic peptide, aromatase inhibitor, 17β-hydroxysteroid dehydrogenase inhibitor, adrenal androgen production inhibitor, kinase inhibitor, drug for hormone therapy such as estrogenes (e.g., DSB, EMP, etc.), antiandrogen (e.g., CMA. etc.), drug inhibiting the action of a cell growth factor or its receptor, and so forth.

For breast cancer, examples of such combination includes compound (I) or a salt thereof in combination with the GnRH super-agonist, antiestrogen, chemotherapeutic agent (e.g., cyclophosphamide, 5-FU, UFT, methotrexate, adriamycin, mitomycin C, mitoxantrone, etc.), GnRH antagonistic peptide, aromatase inhibitor, adrenal androgen production inhibitor, kinase inhibitor, drug for hormone therapy such as antiestrogen (e.g., tamoxifen, etc.), progesterons (e.g., MPA, etc.), androgens, estrogens, etc., drug inhibiting the action of a cell growth factor or its receptor, or the like.

The administration mode of compound (I) or a salt thereof and a concomitant medicament are not particularly limited, provided that compound (I) or a salt thereof and the concomitant medicament are combined upon administration. Such an administration mode may, for example, be (1) an administration of a single formulation obtained by formulating compound (I) or a salt thereof and a concomitant medicament simultaneously, (2) a simultaneous administration via an identical route of two formulations obtained by formulating compound (I) or a salt thereof and a concomitant medicament separately, (3) a sequential and intermittent administration via an identical route of two formulations obtained by formulating compound (I) or a salt thereof and a concomitant medicament separately, (4) a simultaneous administration via different routes of two formulations obtained by formulating compound (I) or a salt thereof and a concomitant medicament separately, (5) a sequential and intermittent administration via different routes of two formulations obtained by formulating compound (I) or a salt thereof and a concomitant medicament separately (e.g., compound (I) or a salt thereof followed by concomitant medicament, or inverse order) and the like.

The preparation of the present invention containing compound (I) or a salt thereof can be used as a prophylactic and/or therapeutic agent for the above-mentioned diseases.

Depending on symptom severity; subject age, sex, weight and sensitivity; duration and intervals of administration; property, dispensing and kind of pharmaceutical preparation; kind of active ingredient etc., daily dose is not subject to limitation. For use in the treatment of the above-described sex hormone-dependent cancers (e.g., prostatic cancer, uterine cancer, breast cancer, pituitary tumor, etc.), prostatic hypertrophy, uterine fibroid, endometriosis, precocious puberty etc., the daily dose of the active ingredient (compound (I) or a salt thereof) in the form of an oral preparation is normally about 0.01 to 30 mg, preferably about 0.02 to 10 mg, and more preferably 0.1 to 10 mg, especially preferably 0.1 to 5 mg per kg weight of mammal, normally in 1 to 4 divided dosages.

The above doses of the active ingredient (compound (I) or a salt thereof) are applicable to the use of the compound of the present invention in the field of animal husbandry or fishery. Daily dose in the form of an oral preparation is about 0.01 to 30 mg, preferably about 0.1 to 10 mg, per kg weight of subject organism, normally in 1 to 3 divided dosages.

The amount of the active ingredient is desirably 0.01 - 90% (W/W), preferably 0.03 - 80% (W/W), more preferably 0.1 - 70% (W/W), relative to the weight of the preparation.

The production methods of the preparation of the present invention are described in the following.

### (1) Preparation comprising active ingredient and organic acid

The preparation can be produced by appropriately blending an active ingredient, an organic acid, an excipient generally used for formulation and the like.
For example, the preparation is obtained by subjecting a blend of an excipient such as lactose, sucrose, mannitol, cornstarch, crystalline cellulose and the like, an active ingredient and an organic acid to production using a binder such as hydroxypropylmethylcellulose, hydroxypropylcellulose, methylcellulose, polyvinyl alcohol, macrogol, pluronic (registered trade mark) F68, gum arabic, gelatin, starch and the like, and adding, where necessary, a disintegrant such as carboxymethylcellulose sodium, calcium carboxymethylcellulose, croscarboxymethylcellulose sodium, polyvinylpyrrolidone, low-substituted hydroxypropylcellulose and the like in a mixer granulator, a wet extrusion-granulator, a fluid bed granulator and the like. As such preparation, preferred is a preparation comprising compound (I) or a salt thereof, and an organic acid.

### (2) Preparation wherein core comprising active ingredient and organic acid is coated with enteric polymer

A core comprising an active ingredient and an organic acid can be produced by coating an inactive carrier as core particle, such as Nonpareil (registered trade mark, manufactured by Freund Corporation), CELPHERE (registered trade mark, manufactured by Asahi Kasei Chemicals Co., Ltd.) and the like, with an appropriate blend of the active ingredient, an organic acid, and an excipient generally used for formulation. For example, the core can be produced by the method described in JP-A-63-301816. When, for example, the core is obtained by applying an active ingredient and the like onto a core particle made of an inactive carrier, for example, by wet granulation using a centrifugal tumbling granulator (CF-mini, CF-360, manufactured by Freund Corporation) or a tumbling granulator (POWREX MP-10) and the like. Alternatively, an active ingredient and the like may be dispersed on the core particle of an inactive carrier and the like for coating while spraying a solution containing a binder and the like.

While the production apparatus is not limited, for example, a centrifugal tumbling granulator and the like are preferably used for the latter coating. Coating by the above-mentioned two kinds of apparatuses may be performed in combination to apply the active ingredient in two steps. When an inactive carrier core is not used, the preparation is obtained by subjecting an excipient such as lactose, sucrose, mannitol, cornstarch, crystalline cellulose and the like, and an active ingredient to production using a binder such as hydroxypropylmethylcellulose, hydroxypropylcellulose, methylcellulose, polyvinyl alcohol, macrogol, pluronic (registered trade mark) F68, gum arabic, gelatin, starch and the like, and adding, where necessary, a disintegrant such as carboxymethylcellulose sodium, calcium carboxymethylcellulose, croscarboxymethylcellulose sodium, polyvinylpyrrolidone, low-substituted hydroxypropylcellulose and the like in a mixer granulator, a wet extrusion-granulator, a fluid bed granulator and the like.

The obtained core is subjected to a sieving operation to give particles having a desired size. The core granules may be prepared by dry granulation using a roller compactor and the like. The size of the particles to be used is 10 µm to 5 mm, preferably 30 µm to 3 mm, more preferably 60 µm to 2 mm. As such preparation, preferred is a preparation wherein a core comprising compound (I) or a salt thereof and an organic acid is coated with an enteric polymer.

For coating of the layer containing the enteric polymer, a core comprising an active ingredient and an organic acid is coated with an appropriate blend of an enteric polymer and an excipient generally used for formulation. For example, a tumbling granulator (POWREX MP-10) and the like can be used for the production. An enteric polymer may be applied singly or in combination of two or more kinds thereof. Alternatively, two or more kinds of polymers may be sequentially applied to form multiple layers.

For coating, an excipient (e.g., hiding agent (titanium oxide and the like) and an antistatic agent (titanium oxide, talc and the like)), which are added as necessary for formulation, may be appropriately added. Furthermore, when a need arises, a plasticizer such as triethyl citrate, dibutyl sebacate, triacetine, diethyl phthalate, polyethylene glycol and the like, a stabilizer and the like may also be used.

The amount of coating is desirably 1 - 200%(W/W), preferably 5 - 100%(W/W), more preferably 10 - 60%(W/W), relative to the core weight.

### (3) Preparation wherein a core comprising active ingredient is coated with a first layer comprising organic acid, and further with a second layer comprising enteric polymer

A core comprising an active ingredient can be produced by coating an inactive carrier as core particle, such as Nonpareil (registered trade mark, manufactured by Freund Corporation), CELPHERE (registered trade mark, manufactured by Asahi Kasei Chemicals Co., Ltd.) and the like, with an appropriate blend of the active ingredient and an excipient generally used for formulation. For example, the core can be produced by the method described in JP-A-63-301816. When, for example, the core is obtained by applying an active ingredient and the like onto a core particle made of an inactive carrier, for example, by wet granulation using a centrifugal tumbling granulator (CF-mini, CF-360, manufactured by Freund Corporation) or a tumbling granulator (POWREX MP-10) and the like. Alternatively, an active ingredient and the like may be dispersed on the core particle of an inactive carrier and the like for coating while spraying a solution containing a binder and the like.

While the production apparatus is not limited, for example, a centrifugal tumbling granulator and the like are preferably used for the latter coating. Coating by the above-mentioned two kinds of apparatuses may be performed in combination to apply the active ingredient in two steps. When an inactive carrier core is not used, the preparation is obtained by subjecting an excipient such as lactose, sucrose, mannitol, cornstarch, crystalline cellulose and the like, and an active ingredient to production using a binder such as hydroxypropylmethylcellulose, hydroxypropylcellulose, methylcellulose, polyvinyl alcohol, macrogol, pluronic (registered trade mark) F68, gum arabic, gelatin, starch and the like, and adding, where necessary, a disintegrant such as carboxymethylcellulose sodium, calcium carboxymethylcellulose, croscarboxymethylcellulose sodium, polyvinylpyrrolidone, low-substituted hydroxypropylcellulose and the like in a mixer granulator, a wet extrusion-granulator, a fluid bed granulator and the like.

The obtained core is subjected to a sieving operation to give particles having a desired size. The core granules may be prepared by dry granulation using a roller compactor and the like. The size of the particles to be used is 10 µm to 5 mm, preferably 30 µm to 3 mm, more preferably 60 µm to 2 mm. As such preparation, preferred is a preparation wherein a core comprising a compound interacting with an enteric polymer is coated with a first layer containing an organic acid, and further with a second layer containing an enteric polymer.

For coating of the layer containing an organic acid, a core containing an active ingredient is coated with an appropriate blend of an organic acid, a coating substance and an excipient generally used for formulation. For example, a tumbling granulator (POWREX MP-10) and the like can be used for the production. A coating substance may be applied singly or in combination of two or more kinds thereof. Alternatively, two or more kinds of coating substances may be sequentially applied to form multiple layers.

Examples of the coating substance include those obtained by appropriately adding saccharides such as sucrose (purified sucrose), starch sugars such as cornstarch and the like, lactose, honey, and sugar alcohols (mannitol, erythritol and the like) and the like to a polymer base such as low-substituted hydroxypropylcellulose, hydroxypropylcellulose, hydroxypropylmethylcellulose, polyvinylpyrrolidone, polyvinyl alcohol, methylcellulose, hydroxyethylmethylcellulose etc. and the like. To a layer containing an organic acid, an excipient (e.g., hiding agent (titanium oxide and the like) and an antistatic agent (titanium oxide, talc and the like)), which are added as necessary for formulation, may be appropriately added besides the above.

The amount of coating is desirably 1 - 300%(W/W), preferably 5 - 200%(W/W), more preferably 10 - 100%(W/W), relative to the core weight.

For coating of the layer containing the enteric polymer, a core produced by coating a core containing an active ingredient with an organic acid is coated with an appropriate blend of an enteric polymer and an excipient generally used for formulation. For example, a tumbling granulator (POWREX MP-10) and the like can be used for the production. An enteric polymer may be applied singly or in combination of two or more kinds thereof. Alternatively, two or more kinds of polymers may be sequentially applied to form multiple layers.

For coating, an excipient (e.g., hiding agent (titanium oxide and the like) and an antistatic agent (titanium oxide, talc and the like)), which are added as necessary for formulation, may be appropriately added. Furthermore, when a need arises, a plasticizer such as triethyl citrate, dibutyl sebacate, triacetine, diethyl phthalate, polyethylene glycol and the like, a stabilizer and the like may also be used.

The amount of coating is desirably 1 - 200%(W/W), preferably 5 - 100%(W/W), more preferably 10 - 60%(W/W), relative to the core weight.

When producing the present preparation, an intermediate coating layer may be formed by coating. For example, when the active ingredient is a medicament unstable to an organic acid, when an organic acid is unstable to an enteric polymer, when an active ingredient is a medicament unstable to an enteric polymer, and the like, it is preferable to form an intermediate coating layer to prevent a direct contact of the active ingredient and the organic acid, thereby increasing the stability of the medicament. Such intermediate coating layer may be formed with multiple layers.

Examples of the coating substance for an intermediate coating layer include those obtained by appropriately adding saccharides such as sucrose (purified sucrose), starch sugars such as cornstarch and the like, lactose, honey, and sugar alcohols (mannitol, erythritol and the like) and the like to a polymer base such as low-substituted hydroxypropylcellulose, hydroxypropylcellulose, hydroxypropylmethylcellulose, polyvinylpyrrolidone, polyvinyl alcohol, methylcellulose, hydroxyethylmethylcellulose etc. and the like. To an intermediate coating layer, an excipient (e.g., hiding agent (titanium oxide and the like) and an antistatic agent (titanium oxide, talc and the like)), which are added as necessary for formulation, may be appropriately added besides the above.

The coating amount of the intermediate coating layer is generally 1 - 150%(W/W), preferably 2 - 100%(W/W), relative to the weight of the core containing an active ingredient, i.e., the main drug. Coating can be performed by a conventional method. For example, the coating layer components of the intermediate layer are preferably diluted with purified water and the like, and sprayed as a liquid. In this case, a binder such as hydroxypropylcellulose and the like is preferably sprayed alongside.

The present invention is explained in more detail in the following by referring to Reference Examples, Examples, Experimental Examples and Test Examples, which are not to be construed as limitative. The additives (sucrose, starch spherical granule, purified sucrose, lactose, cornstarch, low-substituted hydroxypropylcellulose, hydroxypropylmethylcellulose 2910, methacrylic acid copolymer S, methacrylic acid copolymer L, mannitol, talc, triethyl citrate, sodium hydrogen carbonate) used in the Examples are the Japanese Pharmacopoeia Fifteenth Edition or Japanese Pharmaceutical Excipients 2003 compatible products. Examples

### Production of enteric granules of N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea (hereinafter to be abbreviated as "compound A")

Elementary granules (hereinafter to be also referred to as active ingredient granules or core granules) of compound A were produced by a layering method (tumbling mixer fluidized bed coating apparatus (SPIR-A-FLOW, manufactured by Freund Corporation)) .
Vacuum dried elementary granules were coated with a solution of Eudragit S100 (methacrylic acid copolymer S) and Eudragit L100 (methacrylic acid copolymer L) dissolved at 3:1, at a solid content weight ratio of 30% of the elementary granules to give enteric granules. The obtained spherical granules were vacuum dried at 40°C for 16 hr and passed through a round sieve to give 710 µm - 1400 µm granules.
TC-5EW (hydroxypropylmethylcellulose 2910)
L-HPC (low-substituted hydroxypropylcellulose)

**Table 1**

| compound A enteric granule formulation | | |
|---|---|---|
| constitution | components | weight (mg) |
| active ingredient granule | Nonpareil 101 (20-24) | 57.5 |
| | compound A | 10 |
| | mannitol | 20 |
| | L-HPC (LH-32) | 7.5 |
| | TC-5EW | 5 |
| | subtotal | 100 |
| enteric film | Eudragit S100 | 22.5 |
| | Eudragit L100 | 7.5 |
| | sterile talc | 15 |
| | triethyl citrate | 3 |
| | total | 148 |

### Preparation method of core granules

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water, and mannitol, L-HPC and compound A were sequentially added while stirring the mixture with a propeller to give a dispersion.
2) The dispersion was passed through a 250 µm round sieve.
3) The dispersion was layered on the core granules by SPIR-A-FLOW.
4) The obtained granules were classified by a round sieve and 0.71 - 1.40 mm fractions were collected.
5) The collected granules were vacuum dried at 40°C for 16 hr.

**Table 2**

| starting materials | formulation (g) |
|---|---|
| Nonpareil 101(20-24) | 138.00 |
| compound A | 24.00 |
| mannitol | 48.00 |
| L-HPC (LH32) | 18.00 |
| TC-5 EW | 12.00 |
| purified water | 578.00 |

### Preparative method of enteric granule 1

### [test operation]

Apparatus used: SPIR-A-FLOW Model Lab (Freund Corporation), Yamato Lab-stirrer LR500B (Yamato Scientific Co., Ltd.)
1) Triethyl citrate was added to ethanol/water mixture, Eudragit S100 and Eudragit L100 were slowly added with stirring for dissolution, sterile talc was added to give a dispersion, and the dispersion was stirred for 30 min or longer.
2) The dispersion was passed through a 250 µm round sieve.
3) The dispersion was layered on the core granules by SPIR-A-FLOW.
4) The obtained granules were classified by a round sieve and 0.71 - 1.40 mm fractions were collected.
5) The collected granules were vacuum dried at 40°C for 16 hr.

**Table 3**

| starting materials | formulation (g) |
|---|---|
| core granules | 100.00 |
| Eudragit S100 | 22.50 |
| Eudragit L100 | 7.50 |
| sterile talc | 15.00 |
| triethyl citrate | 3.00 |
| EtOH | 388.80 |
| purified water | 43.20 |

### Production of compound A and citric acid-added enteric granules (enteric granule 2)

Since compound A containing active ingredient granules (the above-mentioned core granules) are rapidly eluted in a solution of pH 4.0, citric acid-added granules were prepared to improve dissolution property in the neutral range. As for formulation, citric acid crystals were coated with Eudragit L100 to give core granules. Coating with Eudragit L100 aims to control elution of citric acid and prevent dissolution of an active ingredient in its layering step. Using the core granules, compound A layering granules were prepared, and coated with a 3:1 mixture (weight ratio) of Eudragit S100 and Eudragit L100 as an enteric polymer.

**Table 4**

| citric acid-added enteric granule formulation | | |
|---|---|---|
| constitution | components | weight (mg) |
| core granule | citric acid (400-800 µm) | 30.3 |
| enteric film | Eudragit L100 | 6.1 |
| | sterile talc | 3.0 |
| | triethyl citrate | 0.6 |
| active ingredient layer | compound A | 30 |
| | mannitol | 12 |
| | L-HPC (LH-32) | 10.8 |
| | TC-5EW | 7.2 |
| | subtotal | 100 |
| enteric film | Eudragit S100 | 22.5 |
| | Eudragit L100 | 7.5 |
| | sterile talc | 15 |
| | triethyl citrate | 3 |
| | total | 148 |

### Coating method of enteric-film to citric acid core granules [test operation]

Apparatus used: SPIR-A-FLOW Model Lab (Freund Corporation), Yamato Lab-stirrer LR500B (Yamato Scientific Co., Ltd.)
1) Triethyl citrate was added to ethanol/water (9/1) mixture, Eudragit L100 was slowly added and dissolved, sterile talc was added, and the mixture was stirred for 30 min or longer to give a dispersion.
2) The dispersion was passed through a 250 µm round sieve.
3) The dispersion was applied onto citric acid crystals by SPIR-A-FLOW.
4) The obtained granules were classified by a round sieve and 0.5 - 1.0 mm fractions were collected.

**Table 5**

| starting materials | formulation (g) |
|---|---|
| citric acid (400-800 µm) | 170.00 |
| Eudragit L100 | 34.00 |
| sterile talc | 17.00 |
| triethyl citrate | 3.40 |
| EtOH | 440.64 |
| purified water | 48.96 |

### Coating method of active ingredient layer onto enteric film-coated citric acid core granules

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water, and mannitol, L-HPC and compound A were sequentially added and dispersed while stirring the mixture with a propeller and the dispersion was stirred for 30 min or longer.
2) The dispersion was passed through a 250 µm round sieve.
3) The dispersion was layered on citric acid core granules by SPIR-A-FLOW.
4) The obtained granules were classified by a round sieve and 0.71 - 1.40 mm fractions were collected.

**Table 6**

| starting materials | formulation (g) |
|---|---|
| citric acid core granules (enteric film-coated) | 96.00 |
| compound A | 72.00 |
| mannitol | 28.80 |
| L-HPC (LH32) | 25.92 |
| TC-5 EW | 17.28 |
| purified water | 816.00 |

### Coating method of enteric film onto active ingredient granules

### [test operation]

Apparatus used: SPIR-A-FLOW Model Lab (Freund Corporation), Yamato Lab-stirrer LR500B (Yamato Scientific Co., Ltd.)
1) Triethyl citrate was added to ethanol/water mixture, Eudragit S100 and Eudragit L100 were slowly added with stirring for dissolution, sterile talc was added to give a dispersion, and the dispersion was stirred for 30 min or longer.
2) The dispersion was passed through a 250 µm round sieve.
3) The dispersion was layered on the active ingredient granules by SPIR-A-FLOW.
4) The obtained granules were classified by a round sieve and 0.71 - 1.40 mm fractions were collected.
5) The collected granules were vacuum dried at 40°C for 16 hr.

**Table 7**

| starting materials | formulation (g) |
|---|---|
| active ingredient granules | 100.00 |
| Eudragit S100 | 22.50 |
| Eudragit L100 | 7.50 |
| sterile talc | 15.00 |
| triethyl citrate | 3.00 |
| EtOH | 388.80 |
| purified water | 43.20 |

### Dog oral absorbability of acid-added enteric granules

The oral absorbability of compound A and citric acid-added enteric granule (enteric granule 2) prepared using enteric granule 1 (acid-free enteric granule) and citric acid crystals as a core in dogs under fasting was evaluated (dose: 20 mg/head). Since the dog oral absorbability of enteric granule 2 increased as compared to that of enteric granule 1, an absorption improvement effect by the addition of acid was confirmed.

**Table 8**

| pharmacokinetics parameter (oral, under fasting) | | | | |
|---|---|---|---|---|
| formulation | Cₘₐₓ (ng/ml) | Tₘₐₓ (h) | AUC₀₋₂₄ₕ (ng·h/ml) | MRT (h) |
| citric acid-added enteric granules S/L=3/1 coating | 18.5 (26.6) | 6.0 (1.6) | 117.1 (82.6) | 8.89 (1.21) |
| citric acid-free enteric granules S/L=3/1 coating | 0.360 (0.070) | 8.5 (3.4) | 4.25 (1.74) | 9.72 (1.33) |

| | | | | |
|---|---|---|---|---|
| mean (standard deviation), n=4 | | | | |

### Production of compound A and fumaric acid-added enteric granules (enteric granule 3)

To further increase the oral absorbability of acid combined enteric granules, a preparation to be layered using fumaric acid powder instead of citric acid crystal as an acid to be used for solubilization was produced.

**Table 9**

| enteric granule formulation wherein fumaric acid layer and active ingredient layer of core granules are separated | | |
|---|---|---|
| | component name | weight (mg) |
| core particle | Nonpareil 101 (24-32) | 20 |
| fumaric acid layer | fumaric acid | 20 |
| | mannitol | - |
| | talc | 5 |
| | L-HPC (LH-32) | 5 |
| | TC-5EW | 5 |
| active ingredient layer | compound A | 20 |
| | mannitol | 7 |
| | L-HPC (LH-32) | 10.8 |
| | TC-5EW | 7.2 |
| enteric layer | Eudragit S100 | 22.5 |
| | Eudragit L100 | 7.5 |
| | triethyl citrate | 3 |
| | sterile talc | 15 |
| | total | 148 |

### Coating method of fumaric acid layer onto core particles

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water, and fumaric acid (fine powder grade), L-HPC and sterile talc were sequentially added with stirring to give a dispersion.
2) The dispersion was passed through a 250 µm round sieve.
3) The dispersion was layered on the core particles by SPIR-A-FLOW.
4) The layered particles were dried therein until the exhaustion temperature reached 40°C.
5) The obtained granules were classified by a round sieve and 0.50 - 1.18 mm fractions were collected.

**Table 10**

| starting materials | formulation (g) |
|---|---|
| Nonpareil 101 (24-32) | 100.00 |
| fumaric acid | 100.00 |
| L-HPC (LH32) | 25.00 |
| sterile talc | 25.00 |
| TC-5 EW | 25.00 |
| purified water | 797.22 |

### Coating method of active ingredient layer onto fumaric acid core granules

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water, and mannitol, L-HPC and compound A were sequentially added and dispersed while stirring the mixture with a propeller and the dispersion was stirred for 30 min or longer.
2) The dispersion was passed through a 250 µm round sieve.
3) The dispersion was layered on the core granules by SPIR-A-FLOW.
4) The granules were dried therein until the exhaustion temperature reached 40°C.
5) The obtained granules were classified by a round sieve and 0.71 - 1.40 mm fractions were collected.

**Table 11**

| starting materials | formulation (g) |
|---|---|
| fumaric acid-coated core granules | 110.00 |
| compound A | 40.00 |
| mannitol | 14.00 |
| L-HPC (LH32) | 21.60 |
| TC-5 EW | 14.40 |
| purified water | 510.00 |

### Coating method of enteric film

### [test operation]

Apparatus used: SPIR-A-FLOW Model Lab (Freund Corporation), Yamato Lab-stirrer LR500B (Yamato Scientific Co., Ltd.)
1) Triethyl citrate was added to ethanol/water mixture, Eudragit S100 and Eudragit L100 were slowly added with stirring for dissolution, sterile talc was added to give a dispersion, and the dispersion was stirred for 30 min or longer.
2) The dispersion was passed through a 250 µm round sieve.
3) The dispersion was layered on the core granules by SPIR-A-FLOW.
4) The granules were dried therein for 10 min.
5) The obtained granules were classified by a round sieve and 0.71 - 1.40 mm fractions were collected.
6) The collected granules were vacuum dried at 40°C for 16 hr.

**Table 12**

| starting materials | formulation (g) |
|---|---|
| core granule | 90.00 |
| Eudragit S100 | 20.25 |
| Eudragit L100 | 6.75 |
| sterile talc | 13.50 |
| triethyl citrate | 2.70 |
| EtOH | 349.92 |
| purified water | 38.88 |

### Dog oral absorbability of enteric granule 3

The oral absorbability of enteric granule 3 in dog under fasting was evaluated.
As a result, the oral absorbability of enteric granule 3 was improved as compared to that of enteric granule 1 and enteric granule 2 (enteric granule with citric acid crystal as a core).

**Table 13**

| pharmacokinetics parameter (oral, 20 mg/dog, under fasting) | | | | |
|---|---|---|---|---|
| formulation | Cₘₐₓ (ng/ml) | Tₘₐₓ (h) | AUC₀₋₂₄ₕ (ng·h/ml) | MRT (h) |
| fumaric acid-added enteric granules S/L=3/1 coating | 36.9 (39.1) | 5.5 (1.9) | 196.8 (186.5) | 8.44 (1.15) |
| citric acid-free enteric granules S/L=3/1 coating | 18.5 (26.6) | 6.0 (1.6) | 117.1 (82.6) | 8.89 (1.21) |
| acid-free enteric granules S/L=3/1 coating | 0.360 (0.070) | 8.5 (3.4) | 4.25 (1.74) | 9.72 (1.33) |

| | | | | |
|---|---|---|---|---|
| mean (standard deviation), n=4 | | | | |

### Production of compound A and fumaric acid-added enteric granules with coated intermediate layer (enteric granule 4)

For stabilization of fumaric acid-added enteric granules, enteric granules coated with TC-5EW and talc as an intermediate layer between fumaric acid layer and active ingredient layer, and between active ingredient layer and enteric polymer layer were produced.
The fumaric acid-added enteric granule formulation of compound A coated with the intermediate layer is shown below.

**Table 14**

| | component name | weight (mg) |
|---|---|---|
| core particles | Nonpareil 101 (24-32) | 20 |
| fumaric acid layer | fumaric acid | 20 |
| | talc | 5 |
| | L-HPC (LH-32) | 5 |
| | TC-5EW | 5 |
| intermediate layer 1 | talc | 5 |
| | TC-5EW | 5 |
| active ingredient layer | compound A | 20 |
| | mannitol | 7 |
| | L-HPC (LH-32) | 10.8 |
| | TC-5EW | 7.2 |
| intermediate layer 2 | talc | 5 |
| | TC-5EW | 5 |
| enteric layer | Eudragit S100 | 7.5 |
| | Eudragit L100 | 2.5 |
| | triethyl citrate | 1 |
| | sterile talc | 5 |
| | total | 136 |

### Coating method of fumaric acid onto core particles

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water, and fumaric acid (fine powder grade), L-HPC and sterile talc were sequentially added with stirring to give a dispersion.
2) The dispersion was passed through a 250 µm round sieve.
3) The dispersion was layered on the core particles by SPIR-A-FLOW.
4) The layered particles were dried therein until the exhaustion temperature reached 40°C.
5) The obtained granules were classified by a round sieve and 0.50 - 1.4 mm fractions were collected.

**Table 15**

| starting materials | formulation (g) |
|---|---|
| Nonpareil 101(24-32) | 100.00 |
| fumaric acid | 25.00 |
| mannitol | 75.00 |
| L-HPC (LH32) | 25.00 |
| sterile talc | 25.00 |
| TC-5 EW | 25.00 |
| purified water | 797.22 |

### Coating method of intermediate layer onto fumaric acid core granules

### [test operation]

1) TC-5EW was dissolved in purified water with stirring and, after dissolution, sterile talc was dispersed with stirring.
2) The dispersion was passed through a 250 µm round sieve.
3) The dispersion was layered on the core granules by SPIR-A-FLOW.
4) The granules were dried therein until the exhaustion temperature reached 40°C.
5) The obtained granules were classified by a round sieve and 0.71 - 1.40 mm fractions were collected.

**Table 16**

| starting materials | formulation (g) |
|---|---|
| fumaric acid core granule | 110.00 |
| talc | 10.00 |
| TC-5 EW | 10.00 |
| purified water | 180.00 |

### Coating method of active ingredient layer onto fumaric acid core granules coated with intermediate layer

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water with stirring, and mannitol, L-HPC and compound A were sequentially added and dispersed with further stirring.
2) The dispersion was passed through a 250 µm round sieve.
3) The dispersion was layered on the fumaric acid core granules by SPIR-A-FLOW.
4) The granules were dried therein until the exhaustion temperature reached 40°C.
5) The obtained granules were classified by a round sieve and 0.71 - 1.4 mm fractions were collected.

**Table 17**

| starting materials | formulation (g) |
|---|---|
| fumaric acid core granule (intermediate layer coated) | 117.00 |
| compound A | 36.00 |
| mannitol | 12.60 |
| L-HPC (LH32) | 19.44 |
| TC-5 EW | 12.96 |
| purified water | 459.00 |

### Coating method of intermediate layer onto active ingredient granules

### [test operation]

Apparatus used: SPIR-A-FLOW Model Lab (Freund Corporation), Yamato Lab-stirrer LR500B (Yamato Scientific Co., Ltd.)
1) TC-5EW was dissolved in purified water with stirring, and sterile talc was dispersed with stirring.
2) The dispersion was passed through a 250 µm round sieve.
3) The dispersion was layered on the active ingredient granules by SPIR-A-FLOW.
4) The granules were dried therein until the exhaustion temperature reached 40°C.
5) The obtained granules were classified by a round sieve and 0.85 - 1.40 mm fractions were collected.

**Table 18**

| starting materials | formulation (g) |
|---|---|
| compound A active ingredient granules | 165.00 |
| talc | 7.50 |
| TC-5 EW | 7.50 |
| purified water | 135.00 |

### Coating method of enteric polymer onto intermediate layer-coated active ingredient granule

### [test operation]

Apparatus used: SPIR-A-FLOW Model Lab (Freund Corporation), Yamato Lab-stirrer LR500B (Yamato Scientific Co., Ltd.)
1) Triethyl citrate was added to ethanol/water mixture, Eudragit S100 and Eudragit L100 were slowly added with stirring for dissolution, sterile talc was added to give a dispersion, and the dispersion was stirred for 30 min or longer.
2) The dispersion was passed through a 250 µm round sieve.
3) The dispersion was layered on the active ingredient granules by SPIR-A-FLOW.
4) The granules were dried therein for 10 min.
5) The obtained granules were classified by a round sieve and 0.85 - 1.4 mm fractions were collected.
5) The collected granules were vacuum dried at 40°C for 16 hr.

**Table 19**

| starting materials | formulation (g) |
|---|---|
| core granule | 90.00 |
| Eudragit S100 | 5.62 |
| Eudragit L100 | 1.87 |
| sterile talc | 3.75 |
| triethyl citrate | 0.75 |
| EtOH | 97.16 |
| purified water | 10.80 |

### Industrial Applicability

Using the method of the present invention, the absorbability of the active ingredient in an enteric polymer-coated preparation can be improved, and the absorption rate of the active ingredient in the body can be increased. Particularly, in an enteric polymer coated preparation containing, as an active ingredient, compound (I) or a salt thereof having a superior gonadotropin releasing hormone antagonizing activity and effective as a prophylactic or therapeutic agent for hormone-dependent diseases, namely, sex hormone-dependent cancers (e.g., prostatic cancer, uterine cancer, breast cancer, pituitary tumor, and the like), prostatic hypertrophy, uterine fibroid, endometriosis, metrofibroma, precocious puberty, amenorrhea syndrome, premenstrual syndrome, multilocular ovary syndrome, polycystic ovary syndrome, acne, alopecia, Alzheimer's disease, and the like; as a pregnancy regulator (e.g., contraceptive, etc.), infertility remedy or menstruation regulator, as a prophylactic or therapeutic agent for irritable bowel syndrome; or as a prophylactic agent for postoperative recurrence of sex hormone-dependent cancer, the absorption rate of the active ingredient by the body can be increased, and a low toxic and highly safe preparation also superior in the stability and pharmacokinetics can be provided.

While some of the embodiments of the present invention have been described in detail in the above, it will, however, be evident for those of ordinary skill in the art that various modifications and changes may be made to the particular embodiments shown without substantially departing from the novel teaching and advantages of the present invention. Such modifications and changes are encompassed in the spirit and scope of the present invention as set forth in the appended claims.

This application is based on a patent application No. 2008-226502 filed in Japan, the contents of which are incorporated in full herein by this reference.

## Claims

1. A preparation with improved absorbability of an active ingredient, comprising, as the active ingredient, a compound represented by the formula (I): 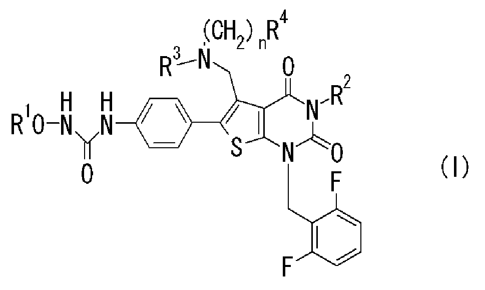 wherein
R¹ is C₁₋₄ alkyl;
R² is
(1) C₁₋₆ alkyl optionally having substituent(s) selected from the group consisting of (1') a hydroxy group, (2') C₁₋₄ alkoxy, (3') C₁₋₄ alkoxy-carbonyl, (4') di-C₁₋₄ alkyl-carbamoyl, (5') a 5- to 7-membered nitrogen-containing heterocyclic group, (6') C₁₋₄ alkyl-carbonyl and (7') halogen,
(2) C₃₋₈ cycloalkyl optionally having (1') a hydroxy group or (2') mono-C₁₋₄ alkyl-carbonylamino,
(3) a 5- to 7-membered nitrogen-containing heterocyclic group optionally having substituent(s) selected from the group consisting of (1') halogen, (2') a hydroxy group, (3') C₁₋₄ alkyl and (4') C₁₋₄ alkoxy,
(4) phenyl optionally having substituent(s) selected from the group consisting of (1') halogen, (2') C₁₋₄ alkoxy-C₁₋₄ alkyl, (3') mono-C₁₋₄ alkyl-carbamoyl-C₁₋₄ alkyl, (4') C₁₋₄ alkoxy and (5') mono-C₁₋₄ alkylcarbamoyl-C₁₋₄ alkoxy or
(5) C₁₋₄ alkoxy;
R³ is C₁₋₄ alkyl;
R⁴ is
(1) a hydrogen atom,
(2) C₁₋₄ alkoxy,
(3) C₆₋₁₀ aryl,
(4) N-C₁₋₄ alkyl-N-C₁₋₄ alkylsulfonylamino,
(5) a hydroxy group or
(6) a 5- to 7-membered nitrogen-containing heterocyclic group optionally having substituent(s) selected from the group consisting of (1') oxo, (2') C₁₋₄ alkyl, (3') hydroxy-C₁₋₄ alkyl, (4') C₁₋₄ alkoxy-carbonyl, (5') mono-C₁₋₄ alkyl-carbamoyl and (6') C₁₋₄ alkylsulfonyl;
n is an integer of 1 to 4;
provided that when R² is phenyl optionally having substituent(s), then R⁴ is a 5- to 7-membered nitrogen-containing heterocyclic group optionally having substituent(s) selected from the group consisting of (1) oxo, (2) hydroxy-C₁₋₄ alkyl, (3) C₁₋₄ alkoxy-carbonyl, (4) mono-C₁₋₄ alkyl-carbamoyl and (5) C₁₋₄ alkylsulfonyl; or a salt thereof, and an organic acid.

2. The preparation according to claim 1, wherein the organic acid is one or more kinds selected from the group consisting of fumaric acid, citric acid, adipic acid, ascorbic acid, benzoic acid, oleic acid, succinic acid, acetic acid, tartaric acid, sorbic acid, lactic acid, maleic acid, malonic acid and malic acid.

3. The preparation according to claim 1, wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea.

4. The preparation according to claim 1, wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea or a salt thereof.

5. The preparation according to claim 1, wherein a core comprising the active ingredient and the organic acid is coated with an enteric polymer.

6. The preparation according to claim 1, wherein a core comprising the active ingredient is coated with a first layer comprising the organic acid, and further with a second layer comprising an enteric polymer.

7. The preparation according to claim 1, wherein the amount of the organic acid is 0.1 - 70%(W/W), and the amount of the active ingredient is 0.01 - 90%(W/W), each relative to the weight of the preparation.

8. A method for improving absorbability of a compound represented by the formula (I): 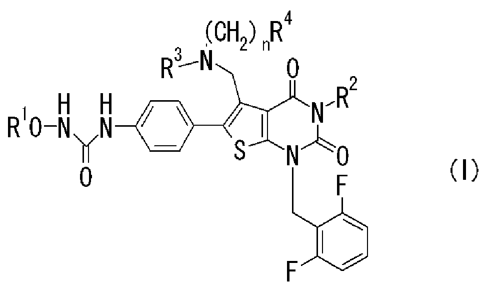 wherein
R¹ is C₁₋₄ alkyl;
R² is
(1) C₁₋₆ alkyl optionally having substituent(s) selected from the group consisting of (1') a hydroxy group, (2') C₁₋₄ alkoxy, (3') C₁₋₄ alkoxy-carbonyl, (4') di-C₁₋₄ alkyl-carbamoyl, (5') a 5- to 7-membered nitrogen-containing heterocyclic group, (6') C₁₋₄ alkyl-carbonyl and (7') halogen,
(2) C₃₋₈ cycloalkyl optionally having (1') a hydroxy group or (2') mono-C₁₋₄ alkyl-carbonylamino,
(3) a 5- to 7-membered nitrogen-containing heterocyclic group optionally having substituent(s) selected from the group consisting of (1') halogen, (2') a hydroxy group, (3') C₁₋₄ alkyl and (4') C₁₋₄ alkoxy,
(4) phenyl optionally having substituent(s) selected from the group consisting of (1') halogen, (2') C₁₋₄ alkoxy-C₁₋₄ alkyl, (3') mono-C₁₋₄ alkyl-carbamoyl-C₁₋₄ alkyl, (4') C₁₋₄ alkoxy and (5') mono-C₁₋₄ alkylcarbamoyl-C₁₋₄ alkoxy or
(5) C₁₋₄ alkoxy;
R³ is C₁₋₄ alkyl;
R⁴ is
(1) a hydrogen atom,
(2) C₁₋₄ alkoxy,
(3) C₆₋₁₀ aryl,
(4) N-C₁₋₄ alkyl-N-C₁₋₄ alkylsulfonylamino,
(5) a hydroxy group or
(6) a 5- to 7-membered nitrogen-containing heterocyclic group optionally having substituent(s) selected from the group consisting of (1') oxo, (2') C₁₋₄ alkyl, (3') hydroxy-C₁₋₄ alkyl, (4') C₁₋₄ alkoxy-carbonyl, (5') mono-C₁₋₄ alkyl-carbamoyl and (6') C₁₋₄ alkylsulfonyl;
n is an integer of 1 to 4;
provided that when R² is phenyl optionally having substituent(s), then R⁴ is a 5- to 7-membered nitrogen-containing heterocyclic group optionally having substituent(s) selected from the group consisting of (1) oxo, (2) hydroxy-C₁₋₄ alkyl, (3) C₁₋₄ alkoxy-carbonyl, (4) mono-C₁₋₄ alkyl-carbamoyl and (5) C₁₋₄ alkylsulfonyl; or a salt thereof, which is an active ingredient of an enteric polymer-coated preparation, which method comprises using an organic acid.

9. The method according to claim 8, wherein the organic acid is one or more kinds selected from the group consisting of fumaric acid, citric acid, adipic acid, ascorbic acid, benzoic acid, oleic acid, succinic acid, acetic acid, tartaric acid, sorbic acid, lactic acid, maleic acid, malonic acid and malic acid.

10. The method according to claim 8, wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea.

11. The method according to claim 8, wherein the active ingredient is N-(4-(1-(2,6-difluorobenzyl)-5-((dimethylamino)methyl)-3-(6-methoxy-3-pyridazinyl)-2,4-dioxo-1,2,3,4-tetrahydrothieno[2,3-d]pyrimidin-6-yl)phenyl)-N'-methoxyurea, or a salt thereof.

12. The method according to claim 8, wherein the enteric polymer-coated preparation is a preparation comprising a core comprising the active ingredient and the organic acid, which is coated with the enteric polymer.

13. The method according to claim 8, wherein the enteric polymer-coated preparation is a preparation comprising a core comprising the active ingredient, which is coated with a first layer comprising the organic acid, and further with a second layer comprising the enteric polymer.
